# FASCICULE DE BREVET EUROPEEN

(11) **EP 0 743 522 B1**
(45) Date de publication et mention de la délivrance du brevet: **12.08.1998**
(21) Numéro de dépôt: 96401041.7
(22) Date de dépôt: 13.05.1996
(51) Int. Cl.: G01N 33/68, C07K 14/47, G01N 33/96

(54) **Composition stabilisée de troponine pour immunoessais et procédé pour sa préparation**
Stabilisierte Troponin-Zubereitung für Immunoassays und Verfahren zur deren Herstellung
Stabilised troponin composition for immunoassays and method for preparing the same

(30) Priorité: 16.05.1995 FR 9505788
(43) Date de publication de la demande: 20.11.1996
(73) Titulaire: PASTEUR SANOFI DIAGNOSTICS, 92430 Marnes La Coquette (FR)
(72) Inventeur: Riochet Denis, Robert, Marie, 92270 Bois Colombes (FR)
(74) Mandataire: Le Guen, Gérard

(56) Documents cités:
- EP-A- 0 650 053
- WO-A-94/27156
- DE-A- 4 243 648
- DE-A- 4 405 249
- GB-A- 2 248 688

## Description

La présente invention concerne une composition stabilisée de troponine pouvant servir d'étalon et/ou de témoin dans les immunoessais visant à doser la ou les troponines cardiaques et/ou squelettiques dans le sérum ou le plasma sanguin de l'homme ou de l'animal, ainsi qu'un procédé de préparation d'une telle composition.

On sait que la troponine est un complexe protéique myofibrillaire, constitué de trois protéines. les troponines I, T et C. Ce complexe protéique permet de contribuer à la régulation de la contraction du muscle par l'ion Ca²⁺, en interagissant avec la myosine et l'actine. De façon plus précise, on sait que lorsqu'un influx nerveux arrive au niveau de la plaque motrice d'un muscle, il y a génération d'un potentiel d'action qui est transmis au réticulum sarcoplasmique. Le Ca²⁺ est alors libéré dans le cytosol et se fixe sur la troponine C, ce qui entraîne un renforcement de l'interaction entre la troponine I et la troponine C et, par suite, un changement de conformation du complexe troponine I,T,C. Il y a alors libération des sites d'interaction actine-myosine, ce qui permet le mouvement de contraction du muscle.

Lorsque le muscle est endommagé, que ce soit le muscle cardiaque lors d'un infarctus du myocarde, ou le muscle squelettique lors d'efforts physiques prolongés, les protéines contractiles alors libérées apparaissent plus ou moins rapidement dans la circulation sanguine.

Ainsi on a récemment préconisé le dosage de la troponine pour le diagnostic précoce de l'infarctus du myocarde, que ce soit celui de la troponine T dans *Circulation 83*, *902-912, (1991)* ou de la troponine I dans *Am. Heart J. 110 1333-44, (1987)* et *Molecular Immunology 29 (2), 271-278 (1992).* De même, on a proposé le dosage de la troponine T cardiaque pour mesurer le succès de la thérapie thrombolytique suite à un infarctus du myocarde dans *Br Heart J 71, 242-248, (1994),* ainsi que le dosage de la troponine I squelettique pour la mesure du dommage musculaire (abrégé n° 35 de *l'American Association for Clinical Chemistry, 46th National Meeting, New Orleans, July 17-21,1994).* Il est à noter que le dosage des différentes troponines cardiaques et squelettiques est aujourd'hui un moyen très utile pour le diagnostic de pathologies humaines et animales.

Il est bien connu que les immunoessais pratiqués dans les laboratoires d'analyses biologiques nécessitent la fourniture par le fabricant, à côté des réactifs nécessaires au dosage (c'est à dire des anticorps, marqués ou non, des agents de révélation et des solutions de dilution), d'un étalon du composé à déterminer qui, mis en oeuvre dans des conditions analogues à celles de l'échantillon à étudier, servira de référence pour le calcul des résultats et/ou de témoin positif.

Pour obtenir l'étalon et/ou le témoin du composé à déterminer, on peut utiliser ledit composé purifié sous forme lyophilisée (accompagné d'un solvant dans lequel le composé sera dissous par l'utilisateur avant l'emploi) ou prêt à l'emploi.

Du fait que les réactifs biologiques sont instables, les solutions étalons ou témoins préparées à partir de lyophilisat sont congelées en dose unitaire et conservées à -80°C. On a constaté par ailleurs que ces solutions n'étaient pas stables plus de quelques heures à +4°C, même si des inhibiteurs de protéases ou des agents antibactériens y étaient ajoutés. Ceci oblige donc les utilisateurs à préparer extemporanément leurs solutions d'étalonnage.

On connaît de l'art antérieur la demande de brevet publiée sous le numéro FR-A- 2 701 954 qui divulgue une composition stabilisée de troponine I ou T pour immunoessai, caractérisée en ce qu'elle est constituée d'une solution aqueuse contenant de la troponine I ou de la troponine T, en mélange avec de la troponine C et notamment dans des proportions de 1 à 10 équivalents molaires de troponine C par équivalent de troponine I ou T, et du CaCl₂. Cette technique permet la conservation durant plusieurs jours à +4°C des solutions étalons, plus ou moins diluées, de troponine I ou T.

La demande de brevet WO 96/33415, cité au titre de l'article 54(3) CBE, décrit (pages 45 et 46) une composition de troponine formée par une solution tamponnée de complexe ternaire troponine I, T et C qui peut contenir des sels de calcium et de magnésium et avoir un pH de 6 à 9.

La présente invention d'une part permet d'obtenir des solutions étalons ou témoins stables durant plusieurs jours à +4°C, et d'autre part présente des avantages considérables tant au niveau du coût de fabrication, que de celui de l'utilisation. Elle permet en effet une utilisation facile et pratique des solutions pour le dosage d'un ou, simultanément ou non, de plusieurs paramètres. La demanderesse a mis en évidence de manière surprenante que le complexe ternaire formé par la troponine I, la troponine T et la troponine C mélangées était stable en solution et que les solutions stabilisées de troponine ainsi obtenues, dont la spécificité et la sensibilité restaient inchangées par rapport à des solutions de composants purifiés, pouvaient être utilisées comme étalon et/ou contrôle dans des tests de diagnostic in vitro d'une ou plusieurs troponines, de façon simultanée si nécessaire.

L'invention a pour objet une composition stabilisée de troponine qui comprend en solution aqueuse de la troponine I. de la troponine T, et de la troponine C et des ions bivalents positifs, les troponines I, T et C étant sous forme de complexe ternaire I-T-C et le pH de la solution étant compris entre 5,5 et 6, la valeur 6 étant exclue.

Les troponines I, T et C peuvent être d'origine humaine ou animale, et peuvent être plus spécifiquement d'origine cardiaque et/ou musculaire.

Les troponines 1, T et C sont obtenues à partir soit d'un extrait de broyat de coeur ou de muscle, soit d'un mélange des trois troponines I, T et C préalablement purifiées.

Il est bien sûr préférable, pour des raisons de coût de fabrication, de préparer les compositions stabilisées de troponines I, T et C selon l'invention à partir d'extrait brut de coeur ou de muscle.

On peut, selon l'origine du mélange des trois troponines, obtenir des quantités plus ou moins variées desdites troponines dans la composition. De façon préférentielle les solutions selon l'invention comprennent une quantité équimolaire de troponine 1, de troponine T et de troponine C, afin d'obtenir une quantité maximale de complexes ternaires I-T-C formés.

Ce complexe ternaire I-T-C est à la base de la stabilité de la composition de troponine selon l'invention. En fonction du procédé de préparation des troponines, on pourra par ailleurs ajouter, pour stabiliser encore plus les protéines entre elles, des ions bivalents positifs, notamment du calcium et du magnésium; qui peuvent être apportés sous forme de chlorure de calcium ou de chlorure de magnésium.

La concentration des troponines I, T et C dans les solutions selon l'invention correspond à celle généralement utilisée dans les immunoessais, c'est à dire qu'elle peut être comprise entre 0,01 ng/ml et 1 µg/ml, et de préférence entre 0,1 ng/ml et 50 ng/ml.

Selon une variante avantageuse, la composition stabilisée selon l'invention comprend de façon préférentielle une concentration de CaCl₂ ou Mg Cl₂ égale à 10² à 5.10⁶ fois en poids celle du complexe de troponine I-T-C, et comprise entre 100 µM et 100 mM. De préférence encore, la composition selon l'invention contient du CaCl₂ 2mM.

Selon une autre variante préférée, la composition stabilisée selon l'invention contient une charge protéique à raison de 0,2 à 2 %, et de préférence de 0,4 à 2 %. Cette charge protéique est constituée par exemple d'albumine bovine, de sérum de veau foetal ou de sérum humain normal. De préférence encore, on utilise du sérum humain normal présent à 10% dans la composition, ce qui correspond à une charge protéique de l'ordre de 0,8 %.

Les tampons que l'on peut utiliser sont par exemple la solution tampon imidazole, la solution tampon phosphate de potassium ou la solution tampon succinate de sodium. On préfèrera utiliser la solution tampon succinate de sodium à 0,1 M.

L'invention a également pour objet une composition stabilisée de troponine en poudre, de préférence sous forme lyophilisée, telle que définie aux revendications 8 et 9.

Les compositions selon l'invention sont utiles pour étalonner et/ou contrôler des tests de diagnostic in vitro de troponine I, de troponine T, ou de troponine C ou de deux ou trois des troponines I, T et C.

L'invention a également pour objet un procédé de préparation d'une composition stabilisée de troponine qui consiste à :
- soit effectuer une extraction de la troponine I, de la troponine T et de la troponine C à partir d'un broyai de coeur ou de muscle humain ou animal, l'extraction se faisant en présence d'anti protéases et d'ions bivalents positifs, notamment du calcium et du magnésium, qui peuvent être apportés sous forme de chlorure de calcium ou de chlorure de magnésium,
- dialyser éventuellement l'extrait obtenu précédemment contre un tampon à pH compris entre 5,5 et 6, la valeur 6 étant exclue, et comprenant des anti protéases et des ions bivalents positifs, notamment du calcium et du magnésium, qui peuvent être apportés sous forme de chlorure de calcium ou de chlorure de magnésium,
- diluer la solution de troponines I, T et C obtenue précédemment dans un tampon à pH avantageusement compris entre 5,5 et 6, la valeur 6 étant exclue, et comprenant des anti protéases, une charge protéique et des ions bivalents positifs, notamment du calcium et du magnésium, qui peuvent être apportés sous forme de chlorure de calcium ou de chlorure de magnésium, afin d'obtenir une concentration appropriée en troponine I, T et/ou C,
- soit mélanger en quantités équimolaires des troponines I, T et C purifiées dans un tampon à pH avantageusement compris entre 5,5 et 6, la valeur 6 étant exclue, et comprenant une charge protéique et des ions bivalents positifs, notamment du calcium et du magnésium, qui peuvent être apportés sous forme de chlorure de calcium ou de chlorure de magnésium.

L'extraction à partir d'un broyat de coeur ou de muscle selon le procédé décrit dans l'invention permet d'obtenir des compositions stables qui peuvent être utilisées pour doser un analyte tel que la troponine I cardiaque ou la troponine T cardiaque, ou utilisées pour doser à partir d'une même composition deux analytes tels que, par exemple, les troponines I cardiaque et T cardiaque.

Dans ce qui suit, on décrit des exemples de réalisation de l'invention et les résultats des essais comparatifs de stabilité.

Le procédé de préparation de compositions stabilisées de troponine selon l'invention est illustré dans les exemples qui suivent. Dans ces exemples, les concentrations, sont exprimées en fonction de la concentration finale de la solution à obtenir.

Préalablement au protocole utilisé pour préparer les compositions stabilisées, on prépare les tampons suivants :
TAMPON D'EXTRACTION :
   - Urée 9 M
   - Tris HCl 75 mM, pH 8
   - CaCl₂ 1mM
   - βMercaptoéthanol 60 mM
   - anti protéases
TAMPON DE DIALYSE :
   - succinate de sodium 0,1M, pH 6
   - CaCl₂ 2 mM
   - anti protéases
TAMPON DE DILUTION :
   - succinate de sodium 0,1 M pH6
   - sérum humain normal (SHN) 10%
   - CaCl₂ 2 mM
   - anti protéases

Les anti protéases utilisées dans les trois tampons précités peuvent être par exemple celles choisies parmi SBTI (Sigma T9003), TLCK (Sigma T7254), Pepstatine A (Sigma P4265), PMSF et l'anticathepsine.

La méthode employée pour effectuer les extractions à partir de coeur ou de muscle est décrite plus en détail dans *American Heart Journal, Clinical Investigations, June 1987, volume 113, n°6,"Cardiac-specific troponin-l radioimmunoassay in the diagnosis of acute myocardial infarction", page 1334.*

### Exemple 1 : Préparation d'une composition de troponine I-T-C cardiaque humaine

On extrait le mélange de troponines 1, T et C à partir d'un gramme de broyat de coeur humain dans 30 ml de tampon d'extraction décrit précédemment. On agite le mélange pendant 15 minutes à l'aide d'un barreau aimanté avant de centrifuger pendant 20 minutes à 10000 g (à +10°C). On récupère le sumageant et on le dialyse contre le tampon de dialyse pendant deux heures, puis pendant une nuit à +4°C en changeant le tampon. On effectue alors une dilution au 1/50^{ème}de la solution obtenue en tampon de dilution.

On obtient une composition comprenant une concentration de 63 ng/ml de troponine l.

### Exemple 2 : Préparation d'une composition de troponine I-T-C cardiaque humaine

On met en oeuvre le même protocole que celui décrit dans l'exemple 1, mais on ne dialyse pas le surnageant après extraction.

On obtient une composition comprenant une concentration de 82 ng/ml de troponine l.

### Exemple 3 : Préparation d'une composition de troponine I-T-C cardiaque humaine

On met en oeuvre le même protocole que celui de l'exemple 1, mais on ajoute, en plus. dans le tampon d'extraction, de dialyse et de dilution du MgCl₂ 4 mM.

On obtient une composition comprenant une concentration de 31 ng/ml de troponine l.

### Exemple 4 : Préparation d'une composition de troponine I-T-C cardiaque humaine

On met en oeuvre le même protocole que celui décrit dans l'exemple 2, mais on ajoute, en plus, dans le tampon d'extraction et de dilution du MgCl₂ 4 mM.On obtient une composition comprenant une concentration de 46 ng/ml de troponine l.

### Exemple 5 : Préparation d'une composition de troponine I-T-C à partir de troponines purifiées

On introduit dans 960 µl de tampon contenant du succinate de sodium (0,1 M, pH 6) contenant 10% de plasma humain normal et 222 µg de CaCl₂, 2H₂O, 10 µl de solution de troponine I à 10 µg/ml, 10 µl de solution de troponine C à 10 µg/ml et 20 µl de solution de troponine T à 5 µg/ml. Il est préférable d'effectuer ces opérations en milieu stérile en utilisant des solutions de troponine l, de troponine T et de troponine C stérilisées, par exemple en leur faisant traverser un filtre de diamètre de pores 0,22 µm.

La solution obtenue de concentration en troponine I voisine de 100 ng/ml, en troponine T de 100 ng/ml et en troponine C de 100 ng/ml est utilisée ensuite pour préparer une gamme de dilutions de 0,1 à 50 ng/ml en troponine I en tampon de conservation (tampon de dilution additionné d'agents anti bactérien à 1% final). Des gammes identiques peuvent être réalisées pour les troponines T et C.

Afin d'effectuer des tests comparatifs de stabilité avec des compositions de troponines purifiées, on répartit les compositions préparées dans les exemples 1 à 4 de la façon suivante:
- stérilement dans des flacons Nalgene® stériles pour les gammes qui seront conservées sous forme de solutions,
- stérilement dans des flacons en verre stériles pour les gammes qui seront lyophilisées.

Des compositions stabilisées de troponine selon l'invention peuvent également être préparées à partir de muscle d'origine humaine, ou de coeur ou de muscle d'origine animale, en utilisant le protocole décrit dans la référence citée précédemment *(American Heart Journal, Clinical Investigations, June 1987, Volume* *113, n°6,"Cardiac-specific troponin-l radioimmunoassay in the diagnosis of acute myocardial infarction", page 1334)*

### Réalisation de tests comparatifs de stabilité :

A partir de solutions concentrées décrites dans les exemples 1 à 4, on prépare des solutions de troponines comprenant une concentration de troponine I de l'ordre de 1 ng/ml. A cet effet, on dilue de façon convenable les solutions dans un tampon de conservation (tampon de dilution additionné de Kathon® 1% final). Le Kathon®, agent anti bactérien commercialisé par la société Haas, est constitué de 5-chloro 2-méthyl 4-isothiazolin-3-one et de 2-méthyl 4-isothiazolin-3-one (1,5%).

Pour chaque exemple, on dispose de flacons liquides et lyophilisés.
- Les flacons lyophilisés sont réhydratés afin de suivre leur stabilité après reconstitution. Le jour 0 (J0) correspond au jour de réhydratation. Dans le tableau I, ces flacons sont nommés Lyoph.liq.
- Les flacons liquides sont conservés à 4°C et suivis en stabilité. Le jour 0 (J0) correspond au jour de préparation. Dans le tableau II, ces flacons sont nommés Liq.

Un contrôle de qualité est effectué avec une référence lyophilisée comprenant de la troponine I purifiée dans du sérum humain normal (noté "Référence" dans le tableau I). Cette référence est préparée et utilisée extemporanément.

Lors de chaque test, les points de gamme préparés sont lus sur une gamme de troponine I lyophilisée telle que celle préparée dans la demande de brevet publiée sous le numéro FR-A- 2 701 954 (5 équivalents molaires de troponine C par équivalent de troponine I et du CaCl₂). A titre comparatif, une solution de troponine I liquide préparée selon l'invention décrite dans la demande de brevet publiée sous le numéro FR-A- 2 701 954, est suivie en stabilité à 4°C. Cette solution comparative est notée "FR-A-2 701 954 " dans les tableaux I et II.

Les tableaux I et II donnent respectivement les résultats de stabilité des compositions selon l'invention, sous forme lyophilisée et liquide, en comparaison avec les compositions des références citées plus haut (les mesures de concentrations sont exprimés en ng/ml).

Le dosage des troponines selon la méthode est décrite dans la demande de brevet FR-A-2 701 954.

A noter que les concentrations initiales des solutions mesurées ne sont pas identiques. De ce fait, pour se prononcer quant à la stabilité, les fluctuations des concentrations observées dans le temps pour la même solution ont été considérées.

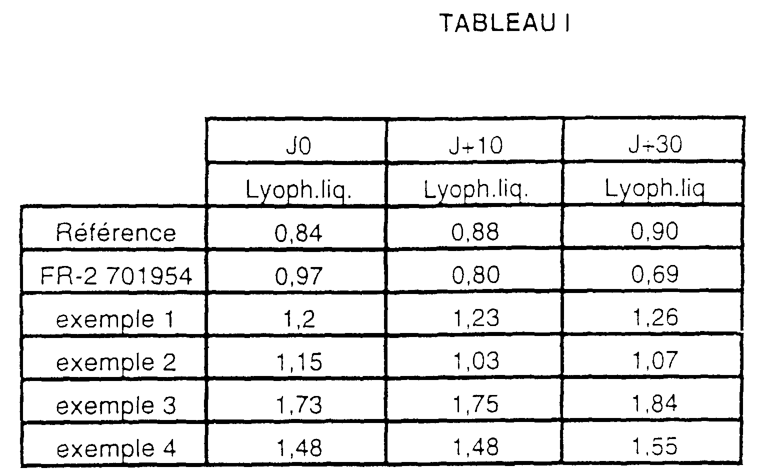

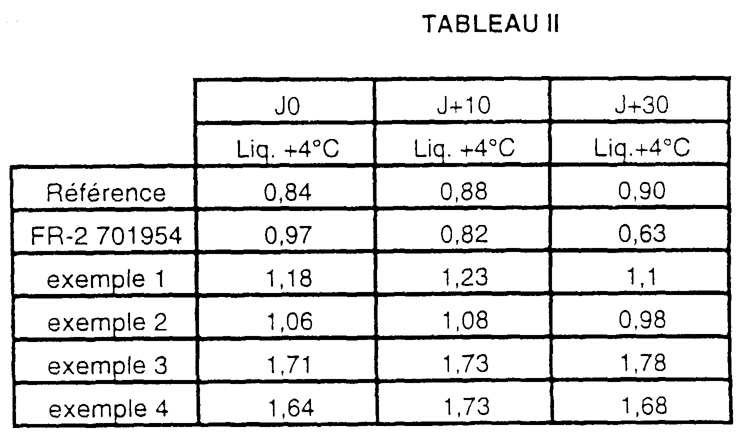

Les résultats des essais effectués démontrent que les compositions des exemples 1 à 4 selon l'invention présentent une stabilité plus élevée à un mois (J+30) par rapport au témoin FR-2 701 954. Les compositions selon l'invention sont donc d'autant plus stables par rapport aux compositions étalons de troponine I purifiée qui ne présentent qu'une stabilité de quelques heures à 4°C. Ces résultats démontrent de façon probante que les compositions de troponine I-T-C selon l'invention présentent une stabilité accrue, et permettent ainsi d'être utilisées en tant qu'étalon et/ou témoin dans des immunoessais visant a doser la ou les troponines cardiaques et /ou squelettiques dans le sérum ou le plasma sanguin de l'homme ou de l'animal.

## Revendications

1. Composition stabilisée de troponine pour immunoessais. caractérisée en ce qu'elle contient en solution aqueuse de la troponine I, de la troponine T, et de la troponine C et des ions bivalents positifs, les troponines I, T et C étant sous forme de complexe ternaire I-T-C et le pH de la solution étant compris entre 5,5 et 6, la valeur 6 étant exclue.

2. Composition selon la revendication 1, caractérisée en ce que le concentration du complexe ternaire I-T-C est comprise entre 0,01 ng/ml et 1 µg/ml, et en ce qu'elle comprend du CaCl₂ à une concentration comprise entre 100 µm et 100 mM.

3. Composition selon l'une quelconque des revendications 1 et 2, caractérisée en ce qu'elle comprend une charge protéique de 0,2 à 2% en poids.

4. Composition selon la revendication 3, caractérisée en ce que la charge protéique est sous forme de sérum humain normal à 10% en volume.

5. Composition selon la revendication 1, caractérisée en ce que la troponine I, la troponine T et la troponine C sont obtenues à partir de l'extraction d'un broyai de coeur humain ou animal.

6. Composition selon la revendication 1, caractérisée en ce que la troponine I, la troponine T et la troponine C sont obtenues à partir de l'extraction d'un broyai de muscle humain ou animal.

7. Composition selon la revendication 5 ou la revendication 6, caractérisée en ce que l'extraction est effectuée en présence de β-mercaptoéthanol.

8. Composition en poudre obtenue par séchage d'une composition aqueuse selon l'une quelconque des revendications précédentes, contenant de la troponine I, de la troponine T, de la troponine C, une charge protéique de 0,2 à 2% et des ions bivalents positifs, ladite composition en poudre présentant, lorsqu'elle est remise en solution, la stabilité de ladite composition aqueuse selon l'une quelconque des revendications précédentes

9. Composition selon la revendication 8, caractérisée en ce qu'elle est sous forme lyophilisée.

10. Utilisation d'une composition selon l'une quelconque des revendications 1 à 9 pour étalonner et/ou contrôler des tests de diagnostic *in* *vitro* de troponine I, de troponine T, ou de troponine C ou de deux ou trois des troponines I, T et C, lesdites troponines pouvant être d'origine cardiaque ou squelettique.

11. Procédé de préparation d'une composition selon l'une quelconque des revendications 1 à 6, caractérisé en ce que :
- on effectue une extraction de la troponine I, de la troponine T ou de la troponine C à partir d'un broyai de coeur ou de muscle humain ou animal, l'extraction se faisant en présence d'anti-protéases et d'ions bivalents positifs;
- on dialyse éventuellement l'extrait obtenu précédemment contre un tampon comprenant des ions bivalents positifs;
- on dilue la solution de troponines I, T et C obtenue précédemment dans un tampon comprenant des anti-protéases, une charge protéique et des ions bivalents positifs, afin d'obtenir une concentration appropriée en troponine I, T et/ou C et un pH compris entre 5,5 et 6, la valeur 6 étant exclue.

12. Procédé selon la revendication 11, caractérisé en ce que l'on effectue l'extraction en présence de β-mercaptoéthanol.

## Patentansprüche

1. Stabilisierte Troponin-Zubereitung für Immunoassays, dadurch gekennzeichnet, daß sie in wässriger Lösung Troponin I, Troponin T und Troponin C und positive zweiwertige Ionen enthält, wobei die Troponine I, T und C in Form des ternären Komplexes I-T-C vorliegen und der pH-Wert der Lösung zwischen 5,5 und 6 liegt, wobei der Wert von 6 ausgeschlossen ist.

2. Zubereitung nach Anspruch 1, dadurch gekennzeichnet, daß die Konzentration des ternären Komplexes I-T-C zwischen 0.01 ng/ml und 1 µg/ml liegt und sie CaCl₂ in einer Konzentration zwischen 100 µm und 100mM enthält.

3. Zubereitung nach einem der Ansprüche 1 und 2, dadurch gekennzeichnet, daß sie einen Proteinzusatz von 0,2 bis 2 Gew.% aufweist.

4. Zubereitung nach Anspruch 3, dadurch gekennzeichnet, daß der Proteinzusatz in Form von normalem menschlichen Serum mit einer Konzentration von 10 Vol.% vorliegt.

5. Zubereitung nach Anspruch 1, dadurch gekennzeichnet, daß Troponin I, Troponin T und Troponin C durch Extraktion von zerkleinerten menschlichen oder tierischen Herzen erhalten worden sind.

6. Zubereitung nach Anspruch 1, dadurch gekennzeichnet, daß Troponin 1, Troponin T und Troponin C durch Extraktion von zerkleinerten menschlichen oder tierischen Muskeln erhalten worden sind.

7. Zubereitung nach Anspruch 5 oder Anspruch 6, dadurch gekennzeichnet, daß die Extraktion in Gegenwart von β-Mercaptoethanol durchgeführt worden ist.

8. Pulverförmige Zubereitung erhalten durch Trocknen einer wässrigen Zubereitung nach einem der vorhergenden Ansprüche, enthaltend Troponin I, Troponin T, Troponin C, einen Proteinzusatz von 0,2 bis 2% und positive zweiwertige Ionen, wobei die pulverförmige Zubereitung, wenn sie erneut in Lösung gebracht worden ist, die Stabilität der wässrigen Zubereitung nach einem der vorhergenden Ansprüche aufweist.

9. Zubereitung nach Anspruch 8, dadurch gekennzeichnet, daß sie in gefriergetrockneter Form vorliegt.

10. Verwendung einer Zubereitung nach einem der Ansprüche 1 bis 9 zur Erzeugung von Eichkurven und/oder zur Überprüfung von in-vitro-Diagnosetests für Troponin I, Troponin T oder Troponin C oder zwei oder drei der Troponine I, T und C, wobei die Troponine vom Herz oder dem Skelett her stammen.

11. Verfahren zur Herstellung einer Zubereitung nach einem der Ansprüche 1 bis 6, dadurch gekennzeichnet, daß man:
- eine Extraktion von Troponin I, Troponin T und Troponin C ausgehend von zerkleinertem menschlichem oder tierischem Herzen oder Muskeln durchführt, wobei die Extraktion in Gegenwart von Antiproteasen und positiven zweiwertigen Ionen erfolgt;
- gegebenenfalls den In der obigen Weise erhaltenen Extrakt gegen einen positive zweiwertige Ionen enthaltenden Puffer dialysiert; und
- die in der obigen Weise erhaltene Lösung der Troponine I, T und C in einem Puffer verdünnt, welcher Antiproteasen, einen Proteinzusatz und positive zweiwertige Ionen enthält, um eine geeignete Konzentration an Troponin I, T und/oder C und einen pH-Wert zwischen 5,5 und 6, wobei der Wert von 6 ausgeschlossen ist, zu erhalten.

12. Verfahren nach Anspruch 11, dadurch gekennzeichnet, daß man die Extraktion in Gegenwart von β-Mercaptoethanol bewirkt.

## Claims

1. Stabilised troponin composition for immunoassays, characterised in that it contains, in aqueous solution, troponin I, troponin T and troponin C and positive bivalent ions, troponins I, T and C being in the form of a ternary I-T-C complex and the pH of the solution being between 5.5 and 6, the value 6 being excluded.

2. Composition according to claim 1, characterised in that the concentration of the ternary I-T-C complex is between 0.01 ng/ml and 1 µg/ml, and in that it contains CaCl₂ in a concentration of between 100 µm and 100 mM.

3. Composition according to one of claims 1 and 2, characterised in that it contains a proteinic charge of 0.2 to 2% by weight.

4. Composition according to claim 3, characterised in that the proteinic charge is in the form of normal human serum at 10% by volume.

5. Composition according to claim 1, characterised in that the troponin I, troponin T and troponin C are obtained by extraction from pulverised human or animal heart.

6. Composition according to claim 1, characterised in that the troponin I, troponin T and troponin C are obtained by extraction from pulverised human or animal muscle.

7. Composition according to claim 5 or 6,
characterised in that the extraction is carried out in the presence of β-mercaptoethanol.

8. Powdered composition obtained by drying an aqueous composition according to any one of the preceding claims, containing troponin I, troponin T, troponin C, a proteinic charge of 0.2 to 2% and positive bivalent ions, said powdered composition, when put into solution, having the same stability as the aqueous composition according to any one of the preceding claims.

9. Composition according to claim 8, characterised in that it is in lyophilised form.

10. Use of a composition according to any one of claims 1 to 9 for calibrating and/or monitoring *in vitro* diagnostic tests on troponin I, troponin T or troponin C or two or three of the troponins I, T and C, said troponins being of cardiac or skeletal origin.

11. Process for preparing a composition according to any one of claims 1 to 6, characterised in that:
troponin I, troponin T and troponin C are extracted from pulverised human or animal heart or muscle, the extraction being carried out in the presence of antiproteases and positive bivalent ions;
the extract obtained previously is optionally dialysed against a buffer containing positive bivalent ions;
the solution of troponins I, T and C obtained previously is diluted in a buffer containing antiproteases, a proteinic charge and positive bivalent ions, in order to obtain a suitable concentration of troponin I, T and/or C and a pH of between 5.5 and 6, the value 6 being excluded.

12. Process according to claim 11, characterised in that the extraction is carried out in the presence of β-mercaptoethanol.
